Europäisches Patentamt

European Patent Office

Office européen des brevets

(11) Numéro de publication : **0 442 776 B1**

# (12) FASCICULE DE BREVET EUROPEEN

(45) Date de publication du fascicule du brevet :
**12.04.95 Bulletin 95/15**

(51) Int. Cl.⁶ : **G01N 33/48**

(21) Numéro de dépôt : **91400257.1**

(22) Date de dépôt : **04.02.91**

(54) **Réactif et méthode d'utilisation de celui-ci pour la numération automatique des leucocytes basophiles du sang en appareils de mesure par variation de résistivité.**

(30) Priorité : **13.02.90 FR 9001660**

(43) Date de publication de la demande :
**21.08.91 Bulletin 91/34**

(45) Mention de la délivrance du brevet :
**12.04.95 Bulletin 95/15**

(84) Etats contractants désignés :
**AT BE CH DE DK ES FR GB GR IT LI LU NL SE**

(56) Documents cités :
**EP-A- 0 177 137
EP-A- 0 316 453
WO-A-84/03771**

(73) Titulaire : **ABX , Société Anonyme dite
Parc Euromédecine,
128, rue du Caducée
F-34184 Montpellier Cédex 4 (FR)**

(72) Inventeur : **Lefevre, Didier
36 rue des Plaisances
F-78200 Mantes la Ville (FR)**
Inventeur : **Champseix, Henri
2 Chemin de Cambas,
Pioch de Baillos
F-34980 Montferrier-sur-Lez (FR)**

(74) Mandataire : **Lhuillier, René et al
Cabinet Lepeudry,
52, avenue Daumesnil
F-75012 Paris (FR)**

EP 0 442 776 B1

## Description

L'invention a pour objet l'analyse de formules sanguines par comptage et discrimination d'au-moins une sous-population leucocytaire et concerne plus particulièrement un réactif et une méthode d'utilisation de celui-ci pour la numération des granulocytes basophiles en cytométrie de flux par mesure de résistivité.

L'importance diagnostique de la détermination précise des différentes population leucocytaires est reconnue depuis longtemps, en effet, l'apparition de rapports leucocytaires anormaux peut être corrélée à l'apparition de diverses maladies.

Les méthodes traditionnelles de coloration spécifique et l'observation au microscope permettaient déjà de distinguer tous les types de leucocytes, c'est à dire les granulocytes (basophiles, éosinophiles et neutrophiles) et les agranulocytes (monocytes et lymphocytes), mais leur mise en oeuvre est très longue.

Divers appareils automatiques sont déjà commercialisés qui permettent le comptage des différentes populations leucocytaires du sang total et divers réactifs et colorants ont été mis au point pour être utilisés dans ces appareils.

Ainsi on peut mesurer les tailles des cellules, après lyse différentielle de leur cytoplasme, soit par mesure de variations de résistivité (comme décrit, par exemple, dans le brevet WO 84/03771) soit par mesure de diffraction optique (comme décrit par exemple dans le brevet US 3.740.143) ; on peut aussi effectuer des colorations (enzymatiques ou non) spécifiques des différents types cellulaires, et particulièrement des trois types de granulocytes, puis mesurer les tailles et densités optiques des cellules à différentes longueurs d'onde (brevet US 3.740.143).

Le comptage des granulocytes basophiles est particulièrement délicat parce que ce type de cellule ne représente que O,5 à 1 % des globules blancs.

Les granulocytes basophiles se distinguent par leur contenu élevé en héparine, un polysaccharide sulfaté qui peut être révélé par des colorants de la famille des phtalocyanures comme le Bleu Alcian ou le Bleu Astra.

La coloration des granulocytes basophiles au Rouge Neutre, classiquement utilisée en méthode manuelle, peut aussi être combinée au traitement de lyse différentielle des leucocytes pour être utilisée en appareil automatique (brevet US 3.740.143).

La demande de brevet européen 0.177.137 décrit un réactif lytique qui permet la destruction progressive de toutes les cellules sanguines à l'exception des basophiles. Ce réactif endommage les membranes cellulaires ce qui entraine la fuite du cytoplasme. A différents intervalles de temps on voit disparaître les différents types cellulaires c'est à dire successivement les érythhrocytes, les lymphocytes, les éosinophiles, les neutrophiles et les monocytes. Après 80 secondes, seuls les basophiles conservent leur taille et leur morphologie normales. Le principe actif de ce réactif est un mélange de surfactant et d'acide dilué. Ce mélange doit être utilisé dans une gamme étroite de pH comprise entre 1,8 et 2,3.

Ce réactif donne de bons résultats dans les appareils de comptage par lecture optique mais il n'est pas adapté aux mesures par résistivité ; en particulier, il pose un problème important d'encrassement de l'orifice de comptage de l'appareil, la destruction des membranes des hématies n'étant pas complète.

Le principe de numération cellulaire par résistivité repose sur le maintien d'un champ électrique où est entretenu un courant constant ; le passage d'une cellule dans ce champ provoque une augmentation de la tension necéssaire au maintien du courant constant (selon la loi d'Ohm). L'impulsion de tension générée par le passage d'une cellule (et qui constitue la mesure enregistrée par l'appareil) est proportionnelle à la résistance opposée, donc au volume de la cellule, indépendamment de sa forme. Ce mode de détection ne s'applique donc qu'à des cellules préalablement traitées (plus particulièrement par cytolyse différentielle) pour obtenir une discrimination très nette des tailles des sous-populations.

Cette méthode est d'un emploi très simple puisqu'il n'est pas nécessaire de colorer les cellules.

La Demanderesse a mis au point une nouvelle formulation du réactif de lyse différentielle qui préserve les granulocytes basophiles pour permettre son utilisation dans les appareils de mesure par résistivité.

Ainsi le réactif selon la présente invention comprend un surfactant de type éther de polyoxyéthylène (comme le Brij® 35 ou 23-lauryl-éther) à une concentration comprise entre 1 et 5 g/l et un mélange d'acide phtalique à une concentration de 0,5 à 1,5 g/l et d'HCl 0,25 mM. Les concentrations relatives des différents constituants sont ajustées pour réaliser un pH compris entre 2,5 et 3,2.

La Demanderesse a constaté qu'on améliorait la sensibilité de la détection en ajoutant au réactif du SDS (dodécyl sulfate de sodium), à une concentration comprise entre 50 et 300 mg/l, comme agent dénaturant des protéines ce qui diminue la formation de dépôts dans l'instrument de mesure ; en outre le SDS accélère la lyse des cellules.

Le réactif sera avantageusement additionné d'un agent antioxydant comme l'hydroxytoluène butylé ou BHT ($C_{11}H_6O$-di-tert-butyl-4-méthylphenol) à une concentration de 10 à 30 mg/l.

Ainsi selon un mode préféré de l'invention, le réactif comprend les constituants suivants :

| | | | | |
|---|---|---|---|---|
| 1 | à | 5 | g/l | de Brij® 35 |
| 0,5 | à | 1,5 | g/l | d'acide phtalique |
| | | 0,25 | mM | HCl |
| 50 | à | 300 | mg/l | de SDS |
| 10 | à | 30 | mg/l | d'hydroxytoluène butylé |

Selon un mode de réalisation de l'invention, le réactif peut encore être additionné de Bleu Astra, colorant spécifique des granules d'héparine, qui assure une meilleure fixation de ceux-ci.

L'invention concerne également une méthode d'utilisation de ce réactif en appareil automatique à cytométrie de flux, par mesure de résistivité, pour la détermination des leucocytes basophiles.

La méthode selon l'invention comprend la mise en contact de l'échantillon sanguin à étudier et du réactif, dans une enceinte thermostatée à une température comprise entre 30 et 40°C pendant une durée de 5 à 30 secondes (la température et le temps de contact étant ajustés, en fonction inverse l'un de l'autre).

La méthode comprend ensuite la détection des cellules intactes, c'est à dire les basophiles, par une mesure de résistivité dans un appareil de détection approprié et l'enregistrement de ces mesures sous forme d'histogramme.

La figure est un histogramme représentatif obtenu par la mise en oeuvre de la méthode selon l'invention sur un échantillon de sang normal.

L'exemple suivant illustre un mode de réalisation de l'invention sans toutefois en limiter la portée.

Exemple

Les concentrations suivantes des divers constituants sont calculées pour la préparation d'un litre de réactif :

| | | |
|---|---|---|
| Brij® 35 | 2,5 | g |
| acide phtalique | 0,9 | g |
| HCl-1N | 0,25 | ml |
| SDS | 200 | mg |
| hydroxytoluène butylé | 25 | mg |
| Eau distillée | ajusté à 1 litre | |

Le pH est maintenu à 2,7 (entre 2,5 et 3,2)

Le réactif peut être additionné de Bleu Astra, à une concentration de 10 à 100 mg/l ; dans les conditions de pH choisies le Bleu Astra est soluble et stable.

Ce réactif peut être utilisé dans tout appareil de comptage automatique des globules sanguins basé sur le principe de la mesure de résistivité [par exemple ABX modèle 504F]. La tension des électrodes est ajustée à 100 V.

L'appareil est réglé pour mettre en contact un échantillon de 15 µl de sang total et 2 ml de réactif. Ce mélange est réalisé dans une enceinte thermostatée à une température de 40°C pendant 17 secondes. (On obtient le même résultat en diminuant la température et en augmentant le temps de contact et vice et versa).

Le réactif provoque une lyse immédiate des globules rouges puis une lyse progressive de tous les autres types de globules à l'exception des granulocytes basophiles. Après 30 secondes de contact à 40°C on peut vérifier par détermination manuelle et observation au microscope que tous les basophiles ont conservé une morphologie intacte. Quand le réactif contient du Bleu Astra, la coloration des basophiles est proportionnelle à la concentration de Bleu utilisée.

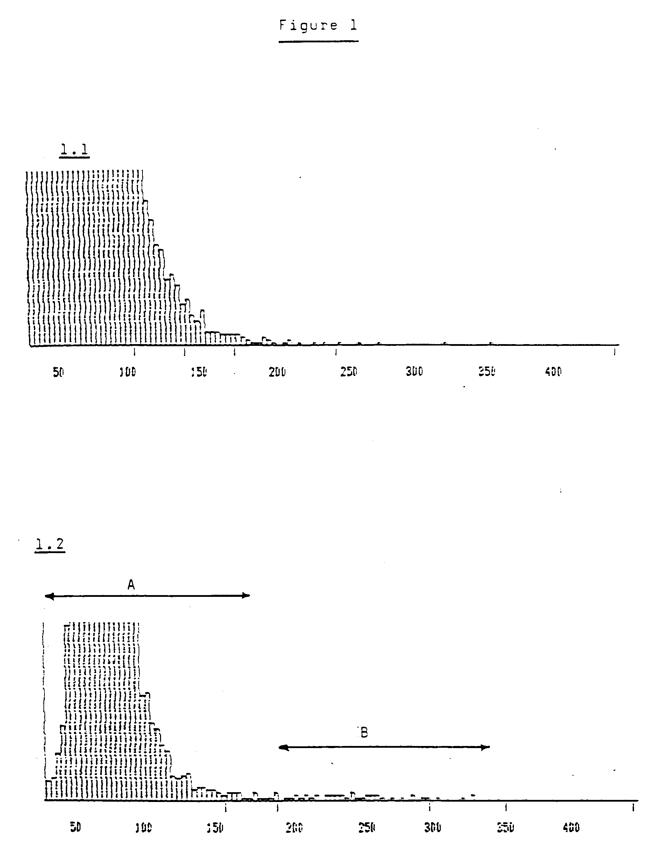

Un histogramme représentatif d'un échantillon de sang normal est présenté sur la figure 1 : la courbe 1.1 représente la détermination effectuée dans du milieu sans SDS ni Bleu Astra et à pH 1,9 ; on observe que les résidus de membrane d'hématies gênent la mesure de résistivité. La courbe 1.2 représente la détermination effectué dans le réactif de la présente invention ; la zone A correspond aux noyaux des cellules lysées ; la zone B correspond aux leucocytes basophiles dont le cytoplasme est intact.

**Revendications**

1. Réactif lytique différentiel pour la numération des leucocytes basophiles du sang dans un analyseur automatique par mesure de résistivité, caractérisé en ce qu'il comprend
   - un surfactant de type éther de polyoxyéthylène
   - un mélange d'acide phtalique - HCl
   - du SDS (dodecylsulfate de sodium)
   - un antioxydant de type hydroxytoluène butylé, en ce que son pH est compris entre 2,5 et 3,2 et qu'il est électriquement conducteur.

2. Réactif selon la revendication 1 caractérisé en ce que le surfactant est du 23-lauryl-éther de polyoxyéthylène à une concentration finale comprise entre 1 et 5 g/l.

3. Réactif selon la revendication 1 ou 2 caractérisé en ce que le mélange d'acide comprend de l'acide phtalique à une concentration finale de 0,5 à 1,5 g/l et de l'HCl à une concentration finale de 0,25 mM.

4. Réactif selon l'une quelconque des revendications 1 à 3 caractérisé en ce qu'il contient du SDS à une concentration finale comprise entre 50 et 300 mg/l.

5. Réactif selon l'une quelconque des revendications 1 à 4 caractérisé en ce qu'il contient de l'hydroxytoluène butylé à une concentration finale de 10 à 30 mg/l.

6. réactif selon l'une quelconque des revendications 1 à 5 caractérisé en ce au'il contient du Bleu Astra à une concentration comprise entre 10 et 100 mg/l.

7. Méthode de numération en appareil automatique en cytométrie de flux de la population de leucocytes basophiles du sang, caractérisée en ce qu'elle comprend :
   - la mise en contact d'un échantillon de sang avec le réactif selon l'une quelconque des revendications 1 à 6, dans une enceinte thermostatée,
   - la détection des cellules intactes c'est à dire des basophiles par mesure de variation de résistivité,
   - l'enregistrement de cette mesure sous forme d'histogramme.

8. Méthode selon la revendication 7 caractérisée en ce que la mise en contact du sang et du réactif est effectuée à une température comprise entre 30 et 40 °C et pendant une durée de 5 à 30 secondes.

**Patentansprüche**

1. Reagens zur differentiellen Lysis für die Zählung von basophilen Leukozyten des Blutes in einer automatischen Analysiervorrichtung durch Messen des Widerstandes, dadurch gekennzeichnet, daß es enthält
   - ein grenzflächenaktives Mittel vom Polyoxyethylenethertyp
   - eine Mischung aus Phtalsäure - HCl
   - SDS (Natriumdodecylsulfat)
   - ein Antioxidans vom butylierten Hydroxytoluoltyp, wobei der pH-Wert des Reagens 2,5 bis 3,2 beträgt und das Reagens einen elektrischen Leiter darstellt.

2. Reagens nach Anspruch 1, dadurch gekennzeichnet, daß das grenzflächenaktive Mittel Polyoxyethylen-23-laurylether in einer Endkonzentration von 1 bis 5 g/l ist.

3. Reagens nach Anspruch 1 oder 2, dadurch gekennzeichnet, daß die Säuremischung Phtalsäure in einer Endkonzentration von 0,5 bis 1,5 g/l und HCl in einer Endkonzentration von 0,25 mM enthält.

4. Reagens nach einem der Vorhergehenden Ansprüche 1 bis 3, dadurch gekennzeichnet, daß es SDS in einer Endkonzentration von 50 bis 300 mg/l enthält.

5. Reagens nach einem der vorhergehenden Ansprüche 1 bis 4, dadurch gekennzeichnet, daß es butyliertes Hydroxytoluol in einer Endkonzentration von 10 bis 30 mg/l enthält.

6. Reagens nach einem der vorhergehenden Ansprüche 1 bis 5, dadurch gekennzeichnet, daß es Astra-Blau in einer Konzentration von 10 bis 100 mg/l enthält.

7. Verfahren zum Zählen der Population von basophilen Leukozyten des Blutes in einer automatischen Vorrichtung zur Fluß-Zytometrie, dadurch gekennzeichnet, daß eine Blutprobe mit dem Reagens nach einem der Ansprüche 1 bis 6 in einer thermostatisierten Umgebung in Kontakt gebracht wird, die intakten Zellen, d.h. die basophilen, durch Messen der Variation des Widerstandes detektiert werden und- diese Messung in Form eines Histogramms aufgezeichnet wird.

8. Verfahren nach Anspruch 7, dadurch gekennzeichnet, daß das Blut mit dem Reagens bei einer Temperatur von 30 bis 40°C und während eines Zeitraumes von 5 bis 30 Sekunden in Kontakt gebracht wird.

## Claims

1. A differential lysing reagent for counting basophilic leucocytes in the blood in an automatic analyser by the measurement of resistivity, characterised in that it comprises
   - a polyoxyethylene ether type surfactant,
   - a phthalic acid-HCl mixture,
   - SDS (sodium dodecyl sulphate)
   - a butylated hydroxytoluene type antioxidant, that its pH is between 2.5 and 3.2, and that it is electrically conductive.

2. A reagent according to claim 1, characterised in that the surfactant is polyoxyethylene-23-lauryl ether at a final concentration between 1 and 5 g/l.

3. A reagent according to claim 1 or 2, characterised in that the acid mixture comprises phthalic acid at a final concentration of 0.5 to 1.5 g/l and HCl at a final concentration of 0.25 mM.

4. A reagent according to any one of claims 1 to 3, characterised in that it contains SDS at a final concentration between 50 and 300 mg/l.

5. A reagent according to any one of claims 1 to 4, characterised in that it contains butylated hydroxytoluene at a final concentration of 10 to 30 mg/l.

6. A reagent according to any one of claims 1 to 5, characterised in that it contains Astra blue at a concentration between 10 and 100 mg/l.

7. A method of counting the population of basophilic leucocytes in the blood in an automatic flow cytometry apparatus, characterised in that it comprises:
   - placing a blood sample in contact with the reagent according to any one of claims 1 to 6, in a thermostatically controlled enclosure,
   - detecting the intact cells, i.e. the basophils, by measuring the variation in resistivity,
   - recording this measurement in the form of a histogram.

8. A method according to claim 7, characterised in that the blood is placed in contact with the reagent at a temperature between 30 and 40°C and for a period of 5 to 30 seconds.

Figure 1

<u>1.1</u>

<u>1.2</u>